# EUROPÄISCHE PATENTSCHRIFT

(11) **EP 1 656 090 B1**
(45) Veröffentlichungstag und Bekanntmachung des Hinweises auf die Patenterteilung: **19.09.2007**
(21) Anmeldenummer: 04763414.2
(22) Anmeldetag: 23.07.2004
(51) Int. Cl.: A61F 13/42, A61F 13/56, A61F 13/15

(54) **WEGWERFWINDEL**
DISPOSABLE NAPPY
COUCHE JETABLE

(30) Priorität: 22.08.2003 DE 10338607
(43) Veröffentlichungstag der Anmeldung: 17.05.2006
(73) Patentinhaber: Paul Hartmann Aktiengesellschaft, 89522 Heidenheim (DE)
(72) Erfinder: RÖHRL, Wolfgang, 89542 Herbrechtingen (DE); STUPPERICH, Hans-Peter, 89522 Heidenheim (DE)
(74) Vertreter: Friz, Oliver
(86) Internationale Anmeldenummer: PCT/EP2004/008219
(87) Internationale Veröffentlichungsnummer: WO 2005/020863

(56) Entgegenhaltungen:
- WO-A-03/007865
- FR-A- 2 585 217
- US-A- 1 695 109
- US-A- 5 569 229
- US-A- 6 102 899
- US-A1- 2002 091 368
- US-B1- 6 605 071

## Beschreibung

Die Erfindung betrifft eine Wegwerfwindel, insbesondere zur Inkontinentenversorgung, mit einem eine in Umfangsrichtung geschlossene Hüftöffnung der Windel bildenden Hüftgürtel, der an wenigstens einer Stelle öffenbar und schließbar ist, und mit einem einen Vorderbereich, einen Rückenbereich und einen dazwischenliegenden Schrittbereich aufweisenden Windelhauptteil, der eine körperabgewandte flüssigkeitsundurchlässige Lage (Backsheet), eine körperzugewandte flüssigkeitsdurchlässige Lage (Topsheet) und einen Absorptionskörper für Körperflüssigkeiten aufweist, wobei der Windelhauptteil mit dem Längsende seines Vorderbereichs oder seines Rückenbereichs über erste Verschlussmittel lösbar am Hüftgürtel festlegbar ist, derart, dass ein Benutzer bei angelegtem Hüftgürtel den Windelhauptteil zwischen den Beinen hervorholen und das noch freie Längsende des Windelhauptteils am Hüftgürtel lösbar festlegen kann.

Derartige Wegwerfwindeln sind vielfach bekannt geworden. Das Dokument WO-A-03007865 offenbart eine derartige windel. Sie bieten den Vorteil, dass ein Benutzer beim Anlegen der Windel zunächst den Hüftgürtel um die Hüften herumlegt, und üblicherweise im Bauchbereich schließen kann. Währenddessen hängt der üblicherweise mit seinem Rückenbereich am Gürtel befestigte Windelhauptteil der Wegwerfwindel lose nach unten. Der Benutzer ergreift dann nach dem Schließen des Hüftgürtels das freihängende Ende des Windelhauptteils und führt den Windelhauptteil von hinten zwischen den Beinen hervor, um den Windelhauptteil mit seinem freien Längsende am Hüftgürtel innen oder außen lösbar festzulegen. Hierfür sind die ersten Verschlussmittel vorgesehen. Es versteht sich, dass das Anlegen der Wegwerfwindel auch so ausgeführt werden kann, dass nach dem Anlegen und Schließen des Hüftgürtels der frei nach unten hängende Windelhauptteil von vorn nach hinten zwischen den Beinen eines Benutzers hindurchgeführt und solchenfalls mit seinem Rückenbereich am Hüftgürtel lösbar befestigt werden kann. Es sind aber auch Wegwerfwindeln bekannt geworden, bei denen der Windelhauptteil ganz vom Hüftgürtel lösbar ist, so dass insbesondere bei stark pflegebedürftigen und immobilen Benutzern eine weitgehende Flexibilität bei der Handhabung der Wegwerfwindel gewährleistet ist.

Hiervon ausgehend liegt der vorliegenden Erfindung die Aufgabe zugrunde, die Verwendbarkeit einer gattungsgemäßen Wegwerfwindel zu erweitern.

Diese Aufgabe wird bei einer Wegwerfwindel der genannten Art erfindungsgemäß dadurch gelöst, dass ausgehend von einem Längsendbereich des Windelhauptteils zwei Sollbruchlinien oder Trennschnitte im Windelhauptteil vorgesehen sind, die sich in Richtung auf den Schrittbereich erstrecken, so dass ein zwischen den Sollbruchlinien oder Trennschnitten angeordneter Mittelabschnitt des Windelhauptteils entlang dieser Sollbruchlinien oder Trennschnitte im Windelhauptteil vom Hüftgürtel weg klappbar oder faltbar ist und wieder mittels zweiter Verschlussmittel mit dem Hüftgürtel und/oder mit angrenzenden Bereichen des Windelhauptteils lösbar verbindbar ist, um die Windel wieder zu schließen.

Die Sollbruchlinien oder die Trennschnitte könnten vom Rand des Längsendbereichs ausgehen oder sie könnten vom Rand beabstandet sein. Im letzten Fall wäre etwa durch eine quer verlaufende Sollbruchlinie oder einen Trennschnitt dafür zu sorgen, dass der Mittelabschnitt herausgelöst und weggeklappt werden kann.

Mit der Erfindung wird also vorgeschlagen, eine Wegwerfwindel, insbesondere zur Versorgung inkontinenter Erwachsener und zur Benutzung durch inkontinente Erwachsene, so auszubilden, dass der Benutzer oder Pflegepersonal die Wegwerfwindel zu Inspektionszwecken wie vorausgehend beschrieben öffnen kann. Dabei bleiben die ersten Verschlussmittel oder zumindest derjenige Teil oder Bereich der ersten Verschlussmittel, welcher den jeweils an die Sollbruchlinien oder Trennschnitte angrenzenden Bereich des Windelhauptteils mit dem Hüftgürtel verbindet, geschlossen. Lediglich die zweiten Verschlussmittel zwischen Windelhauptteil und Hüftgürtel werden geöffnet, oder sie sind ausgehend von der Abgabe der Wegwerfwindel an den Verbraucher, also ausgehend vom verpackten Zustand vor der Ingebrauchnahme in nicht geschlossenem Zustand, vorzugsweise aber in gebrauchsbereitem Zustand. Der Mittelabschnitt des Windelhauptteils, der durch die Sollbruchlinien oder Trennschnitte begrenzt oder definiert wird, kann dann vom Hüftgürtel weg geklappt oder gefaltet werden, um eine Einsichtnahme in das Innere der Windel zu gestatten. Es kann somit geprüft werden, ob die Windel schon verunreinigt, also gebraucht ist, oder noch für die Aufnahme von flüssigen oder festen Körperausscheidungen weiter benutzt werden kann. Es erweist sich als ganz besonders vorteilhaft, dass die Windel hierzu nur durch Lösen eines Teils der Verschlussmittel teilweise geöffnet werden muss, ansonsten aber im bestimmungsgemäß angelegten Zustand am Körper verbleibt. Durch die stützende Funktion der im geschlossenen Zustand verbleibenden Verschlussmittel wird die Wegwerfwindel weiter in der beim Anlegen gewählten optimalen Positionierung am Körper des Benutzers gehalten. Eine Inspektion der Windel ist daher sehr rasch und für den Benutzer wenig störend ausführbar.

Die erfindungsgemäße Wegwerfwindel bietet aber auch einen weiteren Vorteil, in dem sie nicht nur als echter Inkontinenzartikel verwendet werden kann, sondern bei Bedarf zur bevorzugten Verwendung bei männlichen Benutzern eher wie ein Unterbekleidungsstück gehandhabt wird, indem die Wegwerfwindel im Vorderbereich teilweise geöffnet wird, ansonsten aber mit geschlossenem Hüftgürtel weiter angelegt bleibt, während der Benutzer Wasser lassen kann. Solchenfalls klappt oder faltet der Benutzer den Mittelabschnitt des Windelhauptteils zumindest soweit vom Hüftgürtel weg, dass er im ansonsten angelegten Zustand der Windel bei geschlossen gehaltenen Verschlussmitteln im übrigen Wasser lassen kann, ohne die Windel zu beaufschlagen. Dieser Vorteil erweist sich als ganz besonders zweckmäßig bei Verwendung der erfindungsgemäßen Wegwerfwindel durch solche Benutzer, bei denen nur zeitweise die Kontrolle über das Wasserlassen beeinträchtigt ist oder die üblicherweise den Drang, Wasser lassen zu müssen, verspüren und eine entsprechende Bequemlichkeit aufsuchen können, sofern diese in unmittelbarer Nähe zur Verfügung steht. Solchenfalls können die Benutzer bei angelegtem Hüftgürtel die zweiten Verschlussmittel lösen und so den Vorderbereich des Windelhauptteils vom Hüftgürtel weg nach unten klappen oder falten, so dass insbesondere männliche Benutzer dann ihr Glied ergreifen und in üblicher Weise zum Wasserlassen nach außen zu führen vermögen, so dass die Wegwerfwindel nicht mit Flüssigkeit beaufschlagt wird.

Die Möglichkeit einer solchen Verwendung einer Wegwerfwindel ist in ihrer Auswirkung auf die Psyche des Benutzers nicht hoch genug einzuschätzen. Der Benutzer verliert dadurch das Gefühl, dass er auf typische Inkontinenzartikel angewiesen sein soll, auch wenn dies teilweise oder in weitgehenderem Maße tatsächlich der Fall sein sollte. Insbesondere im Kontakt zwischen Arzt oder Pflegepersonal und Patient erleichtert die erfindungsgemäße Ausbildung der Wegwerfwindel die Kommunikation und die Akzeptanz der auferlegten oder angeratenen Verwendung von Inkontinenzartikeln der hier in Rede stehenden Art.

Mit der Erfindung wird also der Vorschlag unterbreitet, durch wenigstens zwei Sollbruchlinien oder Trennschnitte einen Abschnitt des Windelhauptteils zu definieren, der vorausgehend als Mittelabschnitt bezeichnet wurde, ohne dass er notwendigerweise exakt mittig oder symmetrisch zur Mitte angeordnet sein muss, und diesen Mittelabschnitt ausgehend vom angelegten Zustand der Wegwerfwindel wegfaltbar oder aufklappbar auszugestalten. Es erweist sich dabei als vorteilhaft, dass dieser Mittelabschnitt bis auf die Sollbruchlinien oder Trennschnitte weiter vorzugsweise einstückig an den übrigen Windelhauptteil angebunden ist und bleibt.

Der Mittelabschnitt soll also eine Inspektionsöffnung der Wegwerfwindel freigeben und im Anschluss wieder verschließen können.

Es hat sich als vorteilhaft erwiesen, wenn der zwischen den Sollbruchlinien oder Trennschnitten angeordnete Mittelabschnitte des Windelhauptteils in Querrichtung eine Breite von 5 - 50 cm, insbesondere von 5 - 40 cm, insbesondere von 5 - 30 cm, insbesondere von 5 - 25 cm, insbesondere von 8 - 25 cm, insbesondere von 10 - 25 cm, insbesondere von 15 - 25 cm aufweist.

In weiterer Ausbildung der Erfindung variiert die Breite des Mittelabschnitts in Längsrichtung der Wegwerfwindel und nimmt ausgehend vom Längsendbereich des Windelhauptteils in Richtung auf den Schrittbereich ab. Hierdurch wird den anatomischen Gegebenheiten Rechnung getragen.

In weiterer Ausbildung der Erfindung wird vorgeschlagen, dass der Mittelabschnitt des Windelhauptteils in Querrichtung eher breit und die nach dem Wegfalten oder Wegklappen des Mittelabschnitts am Körper verbleibenden an die Sollbruchlinien oder Trennschnitte angrenzenden Bereiche des Windelhauptteils eher schmal ausgebildet sind. Die letzteren angrenzenden Bereiche des Windelhauptteils weisen vorteilhafterweise in Querrichtung eine Breite von 1 - 10 cm, insbesondere von 1 - 8 cm, insbesondere von 1 - 7 cm, insbesondere von 1 - 6 cm, insbesondere von 1 - 5 cm, insbesondere von 2 - 5 cm, insbesondere von 3 - 5 cm auf.

Sofern der wegfaltbare oder wegklappbare Mittelabschnitt des Windelhauptteils lediglich zu Inspektionszwecken verwendet werden soll, erweist es sich als hinreichend wenn die Sollbruchlinien oder Trennschnitte und damit die Längserstreckung des wegfaltbaren oder wegklappbaren Mittelabschnitts eine Länge von etwa 10, insbesondere 10 bis 25 cm aufweist. Wenn der wegklappbare oder wegfaltbare Mittelabschnitt aber auch im eingangs beschriebenen Sinne zur Erleichterung des Wasserlassens im ansonsten angelegten Zustand der Wegwerfwindel verwendet werden soll, so erweist es sich als vorteilhaft, wenn die Sollbruchlinien oder Trennschnitte in Längsrichtung eine Länge von 10 - 60 cm, insbesondere von 10 - 50 cm, insbesondere von 10 - 40 cm, insbesondere von 15 - 40 cm, insbesondere von 20 - 40 cm aufweisen.

Um die Stabilität der Wegwerfwindel und insbesondere die stützende Funktion der im weggeklappten oder weggefalteten Zustand des Windelhauptteils am Körper verbleibenden Bereiche der Windel soweit wie möglich zu erhalten, erweist es sich als vorteilhaft, wenn die Sollbruchlinien oder Trennschnitte in Längsrichtung einen Abstand von wenigstens 1 cm, insbesondere von wenigstens 2 cm, insbesondere von wenigstens 3 cm, insbesondere von wenigstens 4 cm, insbesondere von wenigstens 5 cm bis vorzugsweise 20 cm, insbesondere bis 10 cm von einer Mittelquerlinie des Windelhauptteils aufweisen.

In Weiterbildung der Erfindung von wesentlicher Bedeutung wird vorgeschlagen, die zweiten Verschlussmittel so auszubilden und anzuordnen, dass sie die Sollbruchlinien oder Trennschnitte im Windelhauptteil überfangen. Die zweiten Verschlussmittel können hierfür insbesondere in Form eines laschen- oder bandförmigen Abschnitts ausgebildet sein, welcher sich über eine Sollbruchlinie oder einen Trennschnitt erstreckt. Wenn in weiterer Ausbildung dieses Erfindungsgedankens die zweiten Verschlussmittel die Sollbruchlinien oder Trennschnitte über deren ganze Länge überfangen, so ist es möglich, dass die Sollbruchlinien oder Trennschnitte visuell kaschiert werden. Dies erweist sich als vorteilhaft, weil die Windel hierdurch ein ansprechendes Aussehen erhält und nicht der Eindruck von Nähten oder störenden Unterbrechungen vermittelt wird. Diese Erfindungsvariante erweist sich aber auch aus rein technischen Erwägungen als besonders vorteilhaft. Die zweiten Verschlussmittel können nämlich solchenfalls den Windelhauptteil im Bereich der Sollbruchlinie verstärken. Dies bedeutet, dass die Sollbruchlinie für den Benutzer leicht auftrennbar ausgebildet sein kann, ohne dass zuvor die Gefahr besteht, dass die Sollbruchlinie unbeabsichtigt aufgetrennt wird, und zwar aufgrund der stützenden und stabilisierenden Wirkung der die Sollbruchlinie überfangenden zweiten Verschlussmittel. In entsprechender Weise kann durch die Anordnung der zweiten Verschlussmittel auch ein im Windelhauptteil angebrachter Trennschnitt stabilisiert werden, das heißt der Windelhauptteil bleibt vermittels der den Trennschnitt überfangenden zweiten Verschlussmittel an den angrenzenden Bereich des Windelhauptteils trotz des Trennschnitts angebunden.

Es versteht sich aber, dass für diese Funktion der Anbindung des Mittelabschnitts an den angrenzenden Bereich des Windelhauptteils bei der Abgabe der Wegwerfwindel an den Verbraucher, insbesondere im verpackten Zustand vor Ingebrauchnahme, beliebige Mittel, insbesondere klebende oder mechanisch haftende Bandabschnitte, Laschen oder Streifen, verwendet werden können. Diese könnten insbesondere zum ersten Wegfalten oder Aufklappen des Mittelabschnitts des Windelhauptteils von der Wegwerfwindel ganz abgelöst und verworfen werden. Es wäre auch denkbar, dass diese Stabilisierungsabschnitte in der vorstehend beschriebenen Weise zunächst vom Windelhauptteil ganz abgelöst werden und dann insbesondere um 90° verdreht zum Wiederverschließen des Mittelabschnitts des Windelhauptteils als zweite Verschlussmittel verwendet werden könnten.

Die zweiten Verschlussmittel, welche dem Wiederschließen des Mittelabschnitts des Windelhauptteils dienen, könnten beispielsweise in dessen Längsendbereich ausgebildet sein, etwa im Bereich der Überlappung mit dem Hüftgürtel. Es könnten hier an sich beliebige klebend oder mechanisch wirkende Verschlussmittel oder Verschlusselemente vorgesehen sein, welche mit einer Innenseite oder Außenseite des Hüftgürtels klebend oder haftend zusammenwirken.

Es erweist sich aber als vorteilhaft, wenn die zweiten Verschlussmittel nicht nur mit dem Hüftgürtel, sondern alternativ oder zusätzlich mit den angrenzenden Bereichen des Windelhauptteils zusammenwirken, um die Trennlinie nach dem ersten Aufklappen oder Wegfalten des Mittelabschnitts des Windelhauptteils wieder zu schließen. Es erweist sich als vorteilhaft, wenn die zweiten Verschlussmittel in Längsrichtung der Windel eine Länge von 3 - 20 cm, insbesondere von 5 - 15 cm, insbesondere von 7 - 15 cm aufweisen.

Es erweist sich des Weiteren als vorteilhaft, wenn die zweiten Verschlussmittel beim bestimmungsgemäßen Auffalten oder Wegfalten des Mittelabschnitts des Windelhauptteils an diesem verbleiben und wenn beim Wiederschließen des Mittelabschnitts die zweiten Verschlusselemente gegen angrenzende Bereiche des Windelhauptteils als Auftreffbereich haftend andrückbar sind.

An dieser Stelle sei erwähnt, dass die ersten, aber auch die zweiten Verschlussmittel für sich genommen bekannte mechanisch wirkende Verschlusselemente, insbesondere ein Haken bzw. Schlaufen aufweisendes Material sein können oder aber klebend wirkende Bereiche aufweisen können. Sowohl klebende als auch mechanisch wirkende Verschlusselemente sind hinreichend bekannt und üblich, so dass eine detaillierte Beschreibung entfallen kann. Es sei nur noch erwähnt, dass die ersten Verschlussmittel, aber auch die zweiten Verschlussmittel und die mit ihnen zusammenwirkenden Gegenverschlusselemente am Windelhauptteil oder am Hüftgürtel entweder so angeordnet sein können, dass es dem Benutzer überlassen bleibt, wo er die Halteverbindung ausbildet oder mit welcher Zugspannung er eine Halteverbindung ausbildet. Es besteht indessen auch die Möglichkeit, die ersten und/oder die zweiten Verschlussmittel über vorpositionierte Gegenverschlusselemente oder Gegenverschlussbereiche am Hüftgürtel oder am Windelhauptteil zu schließen. Auf diese Weise wird vom Benutzer eine weitestgehend korrekte Positionierung der Verschlussmittel erzwungen. Solchenfalls erweist sich eine elastisch nachgiebige Ausbildung der eingesetzten Materialien, insbesondere der Verschlussmittel, als vorteilhaft, um verschiedene Körpergrößen und Körpergestalten der Benutzer akkomodieren zu können.

In noch weiterer Ausbildung der Erfindung wird vorgeschlagen, dass die zweiten Verschlussmittel bei der Abgabe an den Verbraucher, insbesondere im verpackten Zustand vor der Ingebrauchnahme, dem Hüftgürtel oder dem Windelhauptteil derart beigeordnet sind, dass sie in dieser Konfiguration auch ungebraucht belassen werden können, ohne störend zu wirken. Sie könnten auch einer Verpackung der Wegwerfwindel losgelöst vom Hüftgürtel und vom Windelhauptteil beigegeben sein. Soll beispielsweise eine erfindungsgemäß ausgebildete Wegwerfwindel ausschließlich als reiner Inkontinenzartikel Verwendung finden oder wird in einer bestimmten Pflegesituation Wert auf ein rasches Anlegen oder Abnehmen des Hygieneartikels, insbesondere durch den Benutzer selbst, gelegt werden, so erweist sich die beschriebene Anordnung oder Beiordnung der zweiten Verschlussmittel als vorteilhaft.

Um den Absorptionskörper des Windelhauptteils mit den tragenden Materialien verbinden zu können, erweist es sich als vorteilhaft, wenn die Sollbruchlinien oder Trennschnitte in Querrichtung der Windel außerhalb des Absorptionskörpers verlaufen, so dass beim Wegfalten oder Wegklappen des Mittelabschnitts des Windelhauptteils der Saugkörper mit weggefaltet wird.

Des Weiteren wird vorgeschlagen, dass die Wegwerfwindel - was für sich genommen bekannt ist - in Längsrichtung erstreckte Elastifizierungsmittel aufweist. Vorzugsweise sind die Elastifizierungsmittel in Querrichtung außerhalb und/oder innerhalb der Sollbruchlinien oder Trennschnitte im Windelhauptteil ausgebildet.

Des Weiteren erweist es sich als ganz besonders vorteilhaft, wenn die ersten und die zweiten Verschlussmittel einander überlappende Bahnabschnitte aufweisen. Solchenfalls können die ersten und die zweiten Verschlussmittel bei der Herstellung in einem einzigen Applikationsvorgang auf den Windelhauptteil appliziert werden. Die ersten und die zweiten Verschlussmittel können als Längsabschnitte einer zuvor in einem vorgeordneten Prozess hergestellten Flachmaterialbahn abgetrennt und appliziert werden.

Nach einer bevorzugten Ausführungsform der Erfindung umfassen die zweiten Verschlussmittel einen die Sollbruchlinie oder den Trennschnitt überfangenden Flachmaterialbahnabschnitt. Sie sind vorzugsweise auf dem Mittelabschnitt festgelegt, insbesondere aufgeklebt. Sie umfassen an ihrem vom Mittelabschnitt in Querrichtung nach außen vorstehenden Bereich eine Klebebeschichtung oder mechanisch wirkende Verschlusselemente, die auf der körperzugewandten Innenseite der Flachmaterialbahnabschnitte vorgesehen sind. Mit diesem Bereich überlappen sie die ebenfalls aus einem Flachmaterialbahnabschnitt gebildeten ersten Verschlussmittel. Die ersten Verschlussmittel bilden im Bereich der Überlappung auf ihrer körperabgewandten Außenseite eine Auftreffzone für die zweiten Verschlussmittel. Die ersten Verschlussmittel umfassen an ihrem vom Windelhauptteil in Querrichtung vorstehenden Abschnitt, und zwar vorzugsweise an der körperzugewandten Innenseite eine Klebebeschichtung oder mechanisch wirkende Verschlusselemente zum Zusammenwirken mit dem Hüftgürtel. Die Längserstreckung der ersten Verschlussmittel beträgt vorzugsweise 1 - 5, insbesondere 1 - 4, insbesondere 1 - 3 cm. Demgegenüber erweist es sich als vorteilhaft, wenn die Längserstreckung der zweiten Verschlussmittel - wie eingangs angedeutet - sich vorzugsweise über die gesamte Längserstreckung der Sollbruchlinie oder des Trennschnitts erstreckt.

Nach einem weiteren für sich genommen selbständigen Erfindungsgedanken, der bei Wegwerfwindeln der gattungsgemäßen Art Anwendung finden kann, sind die in Querrichtung vom Windelhauptkörper sich wegerstreckenden Abschnitte des Hüftgürtels insbesondere leporelloförmig (Z-förmig) auf sich selbst und/oder in Richtung auf eine Längsmittelachse der Wegwerfwindel gefaltet. In Weiterbildung dieses selbständigen Erfindungsgedankens sind die sich beidseits vom Windelhauptkörper wegerstreckenden Abschnitte des Hüftgürtels bei der Abgabe an den Verbraucher, insbesondere im verpackten Zustand vor der Ingebrauchnahme, einander überlappend in Richtung auf die Längsmittelachse der Windel gefaltet. Dabei erstrecken sich ihre freien Enden vorzugsweise nach Außen, also von der Windellängsachse weg, damit sie durch einen Benutzer der Wegwerfwindel gut greifbar sind und der Hüftgürtel infolge des Ergreifens entfaltbar ist.

In weiterer Ausbildung dieses Erfindungsgedankens ist der Hüftgürtel von einem einzigen sich in Umfangsrichtung erstreckenden Materialabschnitt gebildet. In noch weiterer Ausbildung des Erfindungsgedankens ist der insbesondere einstückig ausgebildete Hüftgürtel derart nach Innen auf den Windelhauptteil gefaltet, dass sich eine Faltung des einen Gürtelabschnitts in eine Faltung des anderen Gürtelabschnitts hineinerstreckt.

Weitere Merkmale, Einzelheiten und Vorteile der Erfindung ergeben sich aus den beigefügten Patentansprüchen und der zeichnerischen Darstellung und nachfolgenden Beschreibung bevorzugter Ausführungsformen der erfindungsgemäßen Wegwerfwindel. In der Zeichnung zeigt:
- Figur 1: eine perspektivische Ansicht einer erfindungsgemäßen Wegwerfwindel im angelegten Zustand;
- Figur 2: eine perspektivische Ansicht der Wegwerfwindel im angelegten Zustand nach Figur 1, wobei ein Mittelabschnitt des Windelhauptteils vom Hüftgürtel weggeklappt oder weggefaltet ist;
- Figur 3: eine Draufsicht auf die Wegwerfwindel nach Figuren 1 und 2 im flachgelegten Zustand;
- Figur 4: eine Draufsicht auf einen Teilbereich des Windelhauptteils nach einer bevorzugten Ausführungsform der Erfindung;
- Figur 5: eine Schnittansicht gesehen in Richtung der Pfeile V-V in Figur 4;
- Figur 6: eine Draufsicht auf einen Teilbereich des Windelhauptteils einer weiteren Ausführungsform der erfindungsgemäßen Wegwerfwindel;
- Figur 7: eine Draufsicht auf einen Teilbereich des Windelhauptteils einer weiteren Ausführungsform der erfindungsgemäßen Wegwerfwindel.

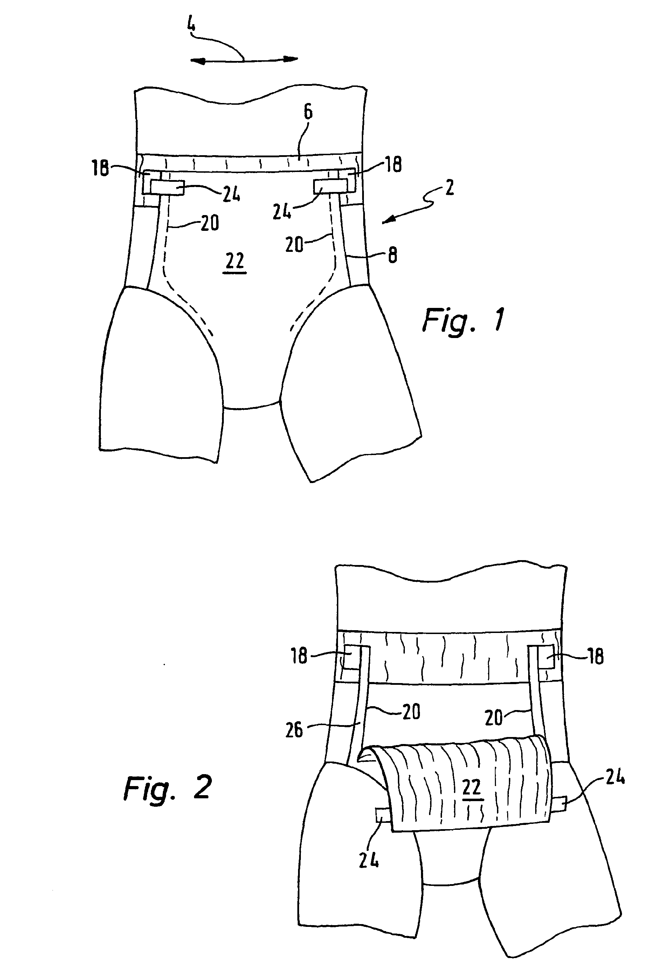

Die Figuren 1 und 2 zeigen eine erfindungsgemäße Wegwerfwindel 2 mit einem in Umfangsrichtung 4 durchgehenden Hüftgürtel 6, der an wenigstens einer Stelle öffenbar bzw. auf sich selbst schließbar ist. Zum Anlegen der Wegwerfwindel 2, legt ein Benutzer den Hüftgürtel 6 um die Hüften und schließt ihn. Sodann holt er den frei hängenden Windelhauptteil 8 zwischen den Beinen hervor und positioniert ihn im Bereich des Bauchs auf der Außenseite des Hüftgürtels 6.

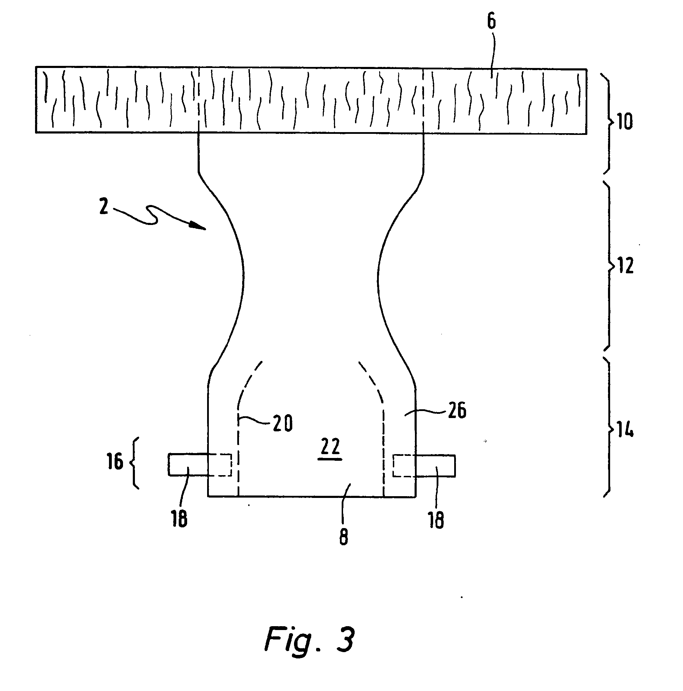

Figur 3 zeigt eine Draufsicht auf die Wegwerfwindel im flachgelegten Zustand. Der Windelhauptteil 8 ist mit seinem Rückenbereich 10, vorzugsweise unlösbar an den Hüftgürtel 6 angefügt. Er umfasst neben dem Rückenbereich 10 einen Schrittbereich 12 und einen Vorderbereich 14. Beim Anlegen der Wegwerfwindel - wie vorstehend beschrieben - überlappt der Windelhauptteil 8 mit einem Längsendbereich 16 seines Vorderbereichs 14 die Außenseite des Hüftgürtels 6. Im dargestellten Fall erstrecken sich in Querrichtung 4 beidseits des Windelhauptteils 8 laschenförmige erste Verschlussmittel 18, mit denen der Windelhauptteil 8 am Hüftgürtel 6 lösbar festlegbar ist.

Man erkennt aus den Figuren 1 bis 3, dass im Vorderbereich 14 des Windelhauptteils 8 Sollbruchlinien 20 oder Trennschnitte vorgesehen sind, die sich ausgehend vom Rand des Längsendbereichs 16 des Vorderbereichs 14 des Windelhauptteils 8 in Richtung auf den Schrittbereich erstrecken. Sie verlaufen - wie beispielhaft dargestellt.-zunächst im wesentlich parallel zueinander und nähern sich dann einander an. Diese Sollbruchlinien 20 sind durch Perforationen gebildet. Sie sind bei der Abgabe der Wegwerfwindel an den Verbraucher zunächst geschlossen. Wie aus Figur 2 ersichtlich ist, können die Sollbruchlinien 20 ausgehend vom Längsendbereich 16 des Windelhauptteils 8 aufgetrennt werden, so dass ein zwischen diesen Sollbruchlinien 20 liegender Mittelabschnitt 22 des Windelhauptteils 8, wie aus Figur 2 ersichtlich, vom Hüftgürtel 6 weggefaltet oder weggeklappt werden kann. Auf diese Weise kann eine Inspektion der angelegten Wegwerfwindel durch Pflegepersonal oder durch den Benutzer selbst vorgenommen werden. Wenn sich die Sollbruchlinien 20 oder alternativ hierzu Trennschnitte weit genug in Richtung auf den Schrittbereich 12 erstrecken, wie dies vorliegend dargestellt ist, so kann der Mittelabschnitt 22 so weit vom Hüftgürtel 6 weggefaltet werden, dass ein Benutzer sein Glied ergreifen und zum Wasserlassen nach außerhalb zu führen vermag. Im Anschluss hieran kann der Mittelabschnitt 22 wieder nach oben gefaltet werden und mittels zweiter Verschlussmittel 24 gegen angrenzende Bereiche 26 des Windelhauptteils 8 lösbar festgelegt werden. Bei diesen angrenzenden Bereichen handelt es sich um die insbesondere aus Figur 2 ersichtlichen am Körper des Benutzers verbleibenden Bereiche des Windelhauptkörpers 8, welche sich jenseits der Sollbruchlinie 20 oder des Trennschnitts befinden und welche über die ersten Verschlussmittel 18 mit dem Hüftgürtel 6 eine lösbare Schließverbindung eingehen.

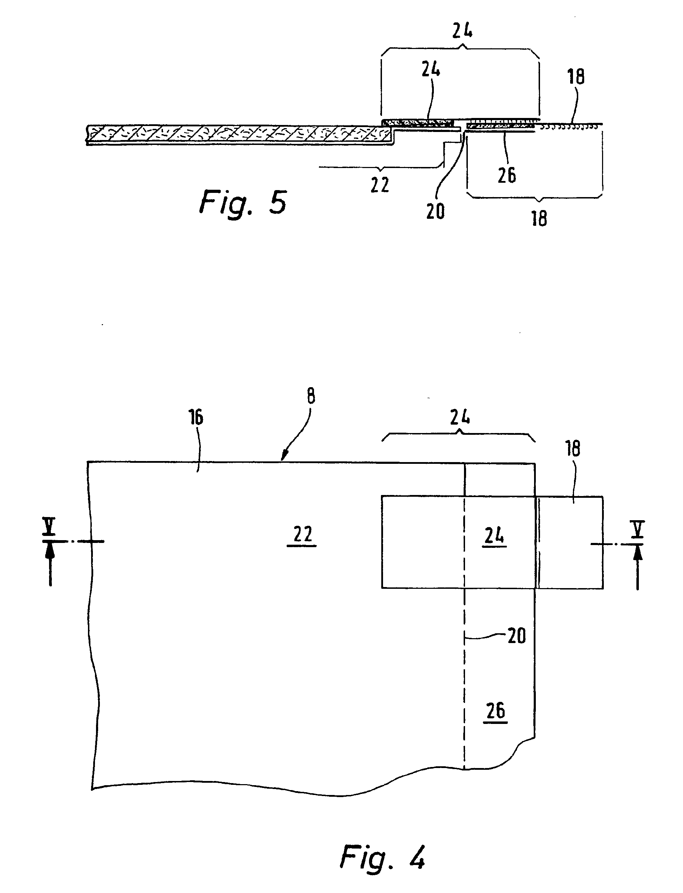

Figur 4 sowie die Schnittdarstellung nach Figur 5 zeigen eine Ausführungsform, bei der die ersten und zweiten Verschlussmittel 18, 24 einander teilweise überlappen.

Man erkennt im dargestellten Fall, dass der an den Mittelabschnitt 22 bzw. die Sollbruchlinie 20 angrenzende Bereich 26 des Windelhauptkörpers 8 streifen- oder stegförmig, also in Längsrichtung langgestreckt ausgebildet ist. Er umfasst eine gegenüber seiner Längserstreckung von etwa 10 bis 50 cm geringere Breite von nur etwa 2 bis 4 cm, insbesondere 2 bis 3 cm, wobei die vorstehenden Angaben rein beispielhaft sind. Die Sollbruchlinie 20 endet in Längsrichtung in einem Abstand von wenigstens 5 cm von einer Mittelquerlinie des Windelhauptteils 8, welche die Längs des Windelhauptteils in zwei gleich lange Teile teilt. Die zweiten Verschlussmittel 24 sind in Form von Tape-Abschnitten oder Laschen ausgebildet, die auf dem wegfaltbaren Mittelabschnitt 22 des Windelhauptteils 8 unlösbar festgelegt sind. Sie erstrecken sich in Umfangs- oder Querrichtung 4 über den seitlichen Rand des Mittelabschnitts 22 hinaus und sind daher in Überdeckung mit dem angrenzenden Bereich 26 des Windelhauptteils 8. Die zweiten Verschlussmittel 24 überdecken sich mit den ebenfalls aus einem Tape oder einer Lasche gebildeten ersten Verschlussmitteln 18, wie dies aus Figur 5 ersichtlich ist. Die Außenseite der ersten Verschlussmittel 18 bildet dabei eine Auftreffzone für die zweiten Verschlussmittel 24. Das erste Verschlussmittel 18 ist unlösbar auf der Außenseite des an den Mittelabschnitt angrenzenden Bereichs 26 festgelegt und dort von dem zweiten Verschlussmittel 24 überfangen. Es erstreckt sich in Umfangs-oder Querrichtung 4 über den Windelhauptteil 8 hinaus und lässt sich mit diesem Bereich in einer Auftreffzone des Hüftgürtels 6 lösbar festlegen.

Der lösbar haftende Bereich der ersten Verschlussmittel 18 und auch der zweiten Verschlussmittel 24 kann in an sich beliebiger Weise klebend oder mechanisch haftend, insbesondere durch ein an sich bekanntes Haken/Schlaufenmaterial, ausgebildet sein.

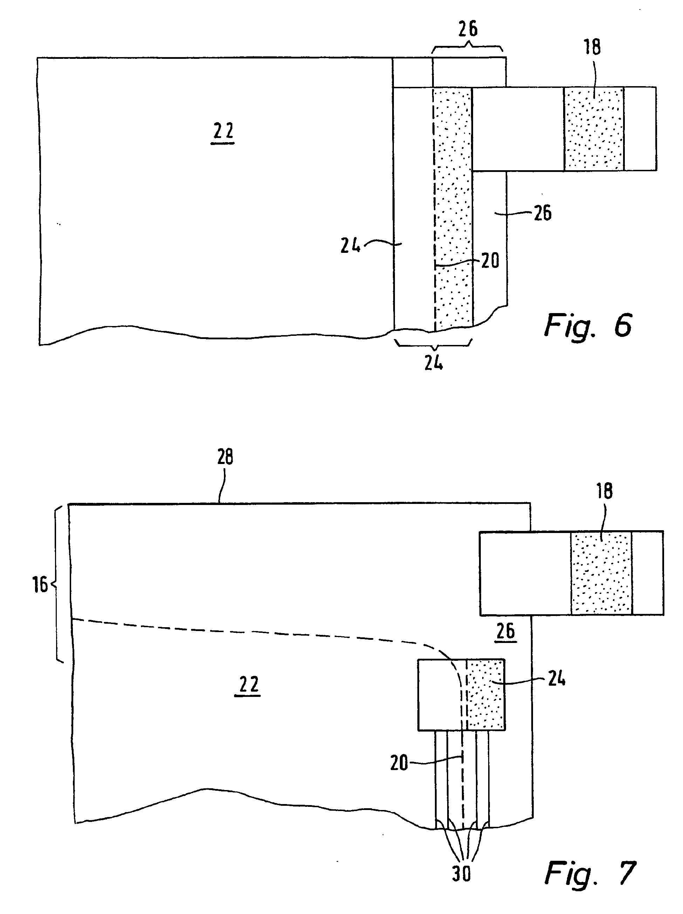

Die Ausführungsform nach Figur 6 unterscheidet sich von derjenigen nach Figuren 4 und 5 dadurch, dass sich die zweiten Verschlussmittel in Längsrichtung über die gesamte Länge der Sollbruchlinie erstrecken und diese dadurch überfangen und visuell kaschieren. Zum Auftrennen der Sollbruchlinien werden die zweiten Verschlussmittel von dem angrenzenden Bereich 26 des Windelhauptteils gelöst um die Sollbruchlinie 20 auftrennen und den Mittelabschnitt 22 des Windelhauptteils - wie aus Figur 2 ersichtlich - aufklappen zu können.

Die Ausführungsform nach Figur 7 unterscheidet sich von den vorhergehenden Ausführungsformen insbesondere darin, dass die Sollbruchlinie 22 nicht vom Rand 28 am Längsende des Vorderbereichs 14 des Windelhauptteils 8 ausgeht, sondern im Längsendbereich 16 in einem Abstand von diesem Rand 28 in Umfangsrichtung 4 nach Innen verläuft. Die beiden den Mittelabschnitt 22 definierenden Sollbruchlinien 20 verlaufen also nach innen und treffen sich dort. Sie bilden die Form eines umgekehrten "U". Im dargestellten Fall der Figur 7 sind die zweiten Verschlussmittel 24 in Längsrichtung von den ersten Verschlussmitteln 18 abgesetzt.

Bei sämtlichen Ausführungsformen verlaufen sowohl innerhalb als auch außerhalb der Sollbruchlinie oder eines Trennschnitts Elastifizierungselemente 30.

## Patentansprüche

1. Wegwerfwindel (2), insbesondere zur Inkontinentenversorgung, mit einem eine in Umfangsrichtung geschlossene Hüftöffnung der Windel bildenden Hüftgürtel (6), der an wenigstens einer Stelle öffenbar und schließbar ist, und mit einem einen Vorderbereich (14), einen Rückenbereich (10) und einen dazwischenliegenden Schrittbereich (12) aufweisenden Windelhauptteil (8), der eine körperabgewandte flüssigkeitsundurchlässige Lage (Backsheet), eine körperzugewandte flüssigkeitsdurchlässige Lage (Topsheet) und einen Absorptionskörper für Körperflüssigkeiten aufweist, wobei der Windelhauptteil (8) mit dem Längsende seines Vorderbereichs (14) oder seines Rückenbereichs (10) über erste Verschlussmittel (18) lösbar am Hüftgürtel (6) festlegbar ist, derart, dass ein Benutzer bei angelegtem.Hüftgürtel (6) den Windelhauptteil (8) zwischen den Beinen hervorholen und das noch freie Längsende des Windelhauptteils (8) am Hüftgürtel (6) lösbar festlegen kann, **dadurch gekennzeichnet, dass** ausgehend von einem Längsendbereich (16) des Windelhauptteils (8) zwei Sollbruchlinien (20) oder Trennschnitte im Windelhauptteil (8) vorgesehen sind, die sich in Richtung auf den Schrittbereich (12) erstrecken, so dass ein zwischen den Sollbruchlinien (20) oder Trennschnitten angeordneter Mittelabschnitt (22) des Windelhauptteils (8) entlang dieser Sollbruchlinien (20) oder Trennschnitte im Windelhauptteil (8) vom Hüftgürtel (6) weg klappbar oder faltbar ist und wieder mittels zweiter Verschlussmittel (24) mit dem Hüftgürtel (6) und/oder mit angrenzenden Bereichen (26) des Windelhauptteils (8) lösbar verbindbar ist, um die Windel wieder zu schließen.

2. Wegwerfwindel nach Anspruch 1, **dadurch gekennzeichnet, dass** der zwischen den Sollbruchlinien (20) oder Trennschnitten angeordnete Mittelabschnitt (22) des Windelhauptteils (8) in Querrichtung (4) eine Breite von 5 - 50 cm, insbesondere von 5 - 40 cm, insbesondere von 5 - 30 cm, insbesondere 5 - 25 cm, insbesondere von 8 - 25 cm, insbesondere von 10 - 25 cm, insbesondere von 15 - 25 cm aufweist.

3. Wegwerfwindel nach Anspruch 1 oder 2, **dadurch gekennzeichnet, dass** die Breite des Mittelabschnitts (22) in Längsrichtung variiert und ausgehend vom Längsendbereich (16) des Windelhauptteils (8) in Richtung auf den Schrittbereich (12) abnimmt.

4. Wegwerfwindel nach Anspruch 1, 2 oder 3, **dadurch gekennzeichnet, dass** die an die Sollbruchlinien (22) oder Trennschnitte angrenzenden Bereiche (26) des Windelhauptteils (8) langgestreckt ausgebildet sind.

5. Wegwerfwindel nach einem oder mehreren der vorstehenden Ansprüche, **dadurch gekennzeichnet, dass** die an die Sollbruchlinien (22) oder Trennschnitte angrenzenden Bereiche (26) des Windelhauptteils (8) in Querrichtung (4) eine Breite von 1 - 10 cm, insbesondere von 1 - 8 cm, insbesondere von 1 - 7 cm, insbesondere von 1 - 6 cm, insbesondere von 1 - 5 cm, insbesondere von 2 - 5 cm, insbesondere von 3 - 5 cm aufweisen.

6. Wegwerfwindel nach einem oder mehreren der vorstehenden Ansprüche, **dadurch gekennzeichnet, dass** die Sollbruchlinien (22) oder Trennschnitte in Längsrichtung eine Länge von 10 - 60 cm, insbesondere von 10 - 50 cm, insbesondere von 10 - 40 cm, insbesondere von 15 - 40 cm, insbesondere von 20 - 40 cm aufweisen.

7. Wegwerfwindel nach einem oder mehreren der vorstehenden Ansprüche, **dadurch gekennzeichnet, dass** die Sollbruchlinien (22) oder Trennschnitte in Längsrichtung einen Abstand von wenigstens 1 cm, insbesondere von wenigstens 2 cm, insbesondere von wenigstens 3 cm, insbesondere von wenigstens 4 cm, insbesondere von wenigstens 5 cm von einer Mittelquerlinie des Windelhauptteils (8) aufweisen.

8. Wegwerfwindel nach einem oder mehreren der vorstehenden Ansprüche, **dadurch gekennzeichnet, dass** die zweiten Verschlussmittel (24) die Sollbruchlinien (22) oder Trennschnitte im Windelhauptteil (8) überfangen.

9. Wegwerfwindel nach Anspruch 8, **dadurch gekennzeichnet, dass** die zweiten Verschlussmittel (24) die Sollbruchlinien (20) oder Trennschnitte über deren ganze Länge überfangen.

10. Wegwerfwindel nach einem oder mehreren der vorstehenden Ansprüche, **dadurch gekennzeichnet, dass** die zweiten Verschlussmittel (24) in Längsrichtung der Windel eine Länge von 3 - 20 cm, insbesondere von 5 - 15 cm, insbesondere von 7 - 15 cm aufweisen.

11. Wegwerfwindel nach einem oder mehreren der vorstehenden Ansprüche, **dadurch gekennzeichnet, dass** die an die Sollbruchlinien (20) oder Trennschnitte angrenzenden Bereiche (26) des Windelhauptteils (8) einen Auftreffbereich für die zweiten Verschlussmittel (24) bilden.

12. Wegwerfwindel nach einem oder mehreren der vorstehenden Ansprüche, **dadurch gekennzeichnet, dass** die Sollbruchlinien (20) durch Schwächung, insbesondere durch Perforation gebildet sind.

13. Wegwerfwindel nach einem oder mehreren der vorstehenden Ansprüche, **dadurch gekennzeichnet, dass** die Sollbruchlinien (20) oder Trennschnitte in Querrichtung (4) der Windel außerhalb des Absorptionskörpers verlaufen.

14. Wegwerfwindel nach einem oder mehreren der vorstehenden Ansprüche, **dadurch gekennzeichnet, dass** in Querrichtung (4)außerhalb und/oder innerhalb der Sollbruchlinien (20) oder Trennschnitte in Längsrichtung verlaufende Elastifizierungsmittel (30) vorgesehen sind.

15. Wegwerfwindel nach einem oder mehreren der vorstehenden Ansprüche, **dadurch gekennzeichnet, dass** die ersten und die zweiten Verschlussmittel (18; 24) einander überlappende Bahnabschnitte aufweisen.

16. Wegwerfwindel nach einem oder mehreren der vorstehenden Ansprüche, **dadurch gekennzeichnet, dass** die ersten Verschlussmittel (18) mit einer körperzugewandten Innenseite gegen eine körperabgewandte Außenseite des Hüftgürtels (6) anlegbar sind.

17. Wegwerfwindel nach einem oder mehreren der vorstehenden Ansprüche, **dadurch gekennzeichnet, dass** die zweiten Verschlussmittel (24) mit einer körperzugewandten Innenseite gegen eine körperabgewandte Außenseite der an die Sollbruchlinien (20) oder Trennschnitte angrenzenden Bereiche (26) des Windelhauptteils (8) anlegbar sind.

## Claims

1. Disposable diaper (2) in particular for incontinent care, with a hip belt (6) for the diaper forming a closed hip opening in a peripheral direction which can be opened and closed at at least one location, and with a main diaper portion (8) having a front region (14), a rear region (10), an intermediate crotch region (12), a liquid-impermeable layer (backsheet) facing away from a body, a liquid-permeable layer (topsheet) facing the body, and an absorption body for bodily fluids, wherein the main diaper portion (8) can be attached to the hip belt (6) in a detachable manner at the longitudinal end of its front region (14) or of its rear region (10) using first closing means (18) and in such a fashion that, with the hip belt (6) applied, a user can raise the main diaper portion (8) between the legs and attach the free longitudinal end of the main diaper portion (8) to the hip belt (6) in a detachable fashion, **characterized in that** two intended breaking lines (20) or separation cuts are provided in the main diaper portion (8) extending from a longitudinal end region (16) of the main diaper portion (8) towards the crotch region (12) in such a fashion that a middle section (22) of the main diaper portion (8), which is disposed between the intended breaking lines (20) or the separation cuts, can be folded away or opened away from the hip belt (6) along the intended breaking lines (20) or separation cuts in the main diaper portion (18) and can be reattached to the hip belt (6) and/or to bordering regions (26) of the main diaper portion (8) using second closing elements (24) to close the diaper again.

2. Disposable diaper according to claim 1, **characterized in that** the middle section (22) of the main diaper portion (8) has a width in the transverse direction (4) between the intended breaking lines (20) or separation cuts of 5 to 50 cm, in particular 5 to 40 cm, in particular 5 to 30 cm, in particular 5 to 25 cm, in particular 8 to 25 cm, in particular 10 to 25 cm, in particular between 15 and 25 cm.

3. Disposable diaper according to claim 1 or 2, **characterized in that** the width of the middle section (22) varies in the longitudinal direction and decreases from the longitudinal end region (16) of the main diaper portion (8) towards the crotch region (12).

4. Disposable diaper according to claim 1, 2 or 3, **characterized in that** the regions (26) of the main diaper portion (8) bordering the intended breaking lines or the separation cuts, are configured in a longitudinally extended fashion.

5. Disposable diaper according to one or more of the preceding claims, **characterized in that** the regions of the main diaper portion (8) which border the intended breaking lines or separation cuts have a width in the transverse direction (4) between 1 to 10 cm, in particular between 1 to 8 cm, in particular between 1 to 7 cm, in particular between 1 to 6 cm, in particular between 1 to 5 cm, in particular between 2 to 5 cm, in particular between 3 to 5 cm.

6. Disposable diaper according to one or more of the preceding claims, **characterized in that** the intended breaking lines or separation cuts have a length in the longitudinal direction of 10 to 60 cm, in particular between 10 to 50 cm, in particular between 10 to 40 cm, in particular between 15 to 40 cm, in particular between 20 to 40 cm.

7. Disposable diaper according to one or more of the preceding claims, **characterized in that** the intended breaking lines or separation cuts are separated in the longitudinal direction from a middle transverse line of the main diaper portion (8) by at least 1 cm, in particular of at least 2 cm, in particular of at least 3 cm, in particular of at least 4 cm, in particular of at least 5 cm.

8. Disposable diaper according to one or more of the preceding claims, **characterized in that** the second closing means (24) bridge over the intended breaking lines or the separation cuts in the main diaper portion (8).

9. Disposable diaper according to claim 8, **characterized in that** the second closing means (24) bridge over the intended breaking lines (20) or separation cuts along their entire length.

10. Disposable diaper according to one or more of the preceding claims, **characterized in that** the second closing means (24) have a length in the longitudinal direction of the diaper of 3 to 20 cm, in particular between 5 to 15 cm, in particular between 7 to 15 cm.

11. Disposable diaper according to one or more of the preceding claims, **characterized in that** the regions (26) of the main diaper portion (8) bordering the intended breaking lines (20) or separation cuts form an impact region for the second closing means (24).

12. Disposable diaper according one ore more of the preceding claims, **characterized in that** the intended breaking lines (20) are formed by weakened locations, in particular perforations.

13. Disposable diaper according to one or more of the preceding claims, **characterized in that** the intended breaking lines (20) or separation cuts extend in a traverse direction (4) of the diaper, outside of the absorption body.

14. Disposable diaper according to one or more of the preceding claims, **characterized in that** elastification means (30) are provided which extend in the longitudinal direction and which are disposed in the transverse direction (4) outside and/or inside the intended breaking lines (20) or the separation cuts.

15. Disposable diaper according to one or more of the preceding claims, **characterized in that** the first and the second closing means (18; 24) have mutually overlapping path sections.

16. Disposable diaper according to one or more of the preceding claims, **characterized in that** the first closing means (18) can be placed, with an inner side facing the body, against an outer side of the hip belt (6) facing away from the body.

17. Disposable diaper according to one or more of the preceding claims, **characterized in that** an inner side of the second closing means (24) facing the body can be placed against an outer side facing away from the body of regions (26) of the main diaper portion (8) bordering the intended breaking lines (20) or the separation cuts.

## Revendications

1. Couche jetable (2), en particulier pour les personnes incontinentes, comportant une ceinture (6) formant une ouverture de taille de la couche fermée dans le sens périphérique, laquelle ceinture peut être ouverte et fermée à au moins un endroit, et comportant une partie principale de couche (8) présentant une zone avant (14), une zone arrière (10) et une zone d'entrejambe (12) placée entre la zone avant et la zone arrière, laquelle partie principale présente une couche imperméable aux liquides orientée à l'opposé du corps (backsheet), une couche perméable aux liquides orientée vers le corps (topsheet) et un corps d'absorption pour les liquides corporels, la partie principale de couche (8) pouvant être fixée de façon détachable à la ceinture (6) avec l'extrémité longitudinale de sa zone avant (14) ou de sa zone arrière (10) par l'intermédiaire de premiers moyens de fermeture (18), de telle sorte qu'un utilisateur, une fois la ceinture (6) mise en place, peut ramener la partie principale de couche (8) entre les jambes et que l'extrémité longitudinale encore libre de la partie principale de couche (8) peut être fixée de façon détachable à la ceinture (6), **caractérisée en ce qu'**à partir d'une zone d'extrémité longitudinale (16) de la partie principale de couche (8), il est prévu deux lignes de rupture (20) ou coupes de séparation dans la partie principale de couche (8), lesquelles s'étendent en direction de la zone d'entrejambe (12) de telle sorte qu'un segment central (22) de la partie principale de couche (8) agencé entre les lignes de rupture (20) ou coupes de séparation peut être rabattu ou plié depuis la ceinture (6) en suivant ces lignes de rupture (20) ou coupes de séparation et peut être de nouveau relié de façon détachable, à l'aide de seconds moyens de fermeture (24), à la ceinture (6) et/ou à des zones (26) adjacentes de la partie principale de couche (8), pour refermer la couche.

2. Couche jetable selon la revendication 1, **caractérisée en ce que** le segment central (22) de la partie principale de couche (8) agencé entre les lignes de rupture (20) ou coupes de séparation présente dans le sens transversal (4) une largeur comprise entre 5 et 50 cm, en particulier entre 5 et 40 cm, en particulier entre 5 et 30 cm, en particulier entre 5 et 25 cm, en particulier entre 8 et 25 cm, en particulier entre 10 et 25 cm, en particulier entre 15 et 25 cm.

3. Couche jetable selon la revendication 1 ou 2, **caractérisée en ce que** la largeur du segment central (22) varie dans le sens longitudinal et diminue à partir de la zone d'extrémité longitudinale (16) de la partie principale de couche (8) en direction de la zone d'entrejambe (12).

4. Couche jetable selon la revendication 1, 2 ou 3, **caractérisée en ce que** les zones (26) de la partie principale de couche (8) adjacentes aux lignes de rupture (20) ou coupes de séparation sont réalisées allongées.

5. Couche jetable selon une ou plusieurs des revendications précédentes, **caractérisée en ce que** les zones (26) de la partie principale de couche (8) adjacentes aux lignes de rupture (20) ou coupes de séparation présentent dans le sens transversal (4) une largeur comprise entre 1 et 10 cm, en particulier entre 1 et 8 cm, en particulier entre 1 et 7 cm, en particulier entre 1 et 6 cm, en particulier entre 1 et 5 cm, en particulier entre 2 et 5 cm, en particulier entre 3 et 5 cm.

6. Couche jetable selon une ou plusieurs des revendications précédentes, **caractérisée en ce que** les lignes de rupture (20) ou coupes de séparation présentent dans le sens longitudinal une longueur comprise entre 10 et 60 cm, en particulier entre 10 et 50 cm, en particulier entre 10 et 40 cm, en particulier entre 15 et 40 cm, en particulier entre 20 et 40 cm.

7. Couche jetable selon une ou plusieurs des revendications précédentes, **caractérisée en ce que** les lignes de rupture (20) ou coupes de séparation présentent dans le sens longitudinal une distance d'au moins 1 cm, en particulier d'au moins 2 cm, en particulier d'au moins 3 cm, en particulier d'au moins 4 cm, en particulier d'au moins 5 cm par rapport à une ligne transversale médiane de la partie principale de couche (8).

8. Couche jetable selon une ou plusieurs des revendications précédentes, **caractérisée en ce que** les seconds moyens de fermeture (24) recouvrent les lignes de rupture (20) ou coupes de séparation dans la partie principale de couche (8).

9. Couche jetable selon la revendication 8, **caractérisée en ce que** les seconds moyens de fermeture (24) recouvrent les lignes de rupture (20) ou coupes de séparation sur toute leur longueur.

10. Couche jetable selon une ou plusieurs des revendications précédentes, **caractérisée en ce que** les seconds moyens de fermeture (24) présentent dans le sens longitudinal de la couche une longueur comprise entre 3 et 20 cm, en particulier entre 5 et 15 cm, en particulier entre 7 et 15 cm.

11. Couche jetable selon une ou plusieurs des revendications précédentes, **caractérisée en ce que** les zones (26) de la partie principale de couche (8) adjacentes aux lignes de rupture (20) ou coupes de séparation forment une zone d'impact pour les seconds moyens de fermeture (24).

12. Couche jetable selon une ou plusieurs des revendications précédentes, **caractérisée en ce que** les lignes de rupture (20) sont formées par affaiblissement, en particulier par perforation.

13. Couche jetable selon une ou plusieurs des revendications précédentes, **caractérisée en ce que** les lignes de rupture (20) ou coupes de séparation s'étendent dans le sens transversal (4) de la couche en dehors du corps d'absorption.

14. Couche jetable selon une ou plusieurs des revendications précédentes, **caractérisée en ce que** des moyens d'élastification (30) s'étendant dans le sens longitudinal sont prévus dans le sens transversal (4) à l'extérieur et/ou à l'intérieur des lignes de rupture (20) ou coupes de séparation.

15. Couche jetable selon une ou plusieurs des revendications précédentes, **caractérisée en ce que** les premiers et seconds moyens de fermeture (18 ; 24) présentent des segments de bande qui se chevauchent.

16. Couche jetable selon une ou plusieurs des revendications précédentes, **caractérisée en ce que** les premiers moyens de fermeture (18) peuvent s'appliquer avec un côté intérieur orienté vers le corps contre un côté extérieur de la ceinture (6) orienté à l'opposé du corps.

17. Couche jetable selon une ou plusieurs des revendications précédentes, **caractérisée en ce que** les seconds moyens de fermeture (24) peuvent s'appliquer avec un côté intérieur orienté vers le corps contre un côté extérieur orienté à l'opposé du corps des zones (26) de la partie principale de couche (8) adjacentes aux lignes de rupture (20) ou coupes de séparation.
